# FASCICULE DE BREVET EUROPEEN

(11) **EP 1 937 185 B1**
(45) Date de publication et mention de la délivrance du brevet: **24.02.2010**
(21) Numéro de dépôt: 06808178.5
(22) Date de dépôt: 21.09.2006
(51) Int. Cl.: A61F 2/20

(54) **IMPLANT PHONATOIRE ET DISPOSITIF D'INSERTION**
IMPLANTIERBARE SPRACHPROTHESE UND IMPLANTATIONSINSTRUMENT
PHONATORY IMPLANT AND DEVICE FOR INSERTING

(30) Priorité: 23.09.2005 FR 0509730
(43) Date de publication de la demande: 02.07.2008
(73) Titulaire: PROTIP SAS, 67380 Lingolsheim (FR); Université Louis Pasteur, U.L.P., 67000 Strasbourg (FR); Hôpitaux Universitaires de Strasbourg (HUS), 67000 Strasbourg (FR)
(72) Inventeur: DEBRY, Christian, F-75015 Paris (FR); WALDER, André, F-94240 L'Hay les Roses (FR)
(74) Mandataire: Barny, Luc
(86) Numéro de dépôt international: PCT/FR2006/002156
(87) Numéro de publication internationale: WO 2007/034077

(56) Documents cités:
- EP-A- 0 222 509
- EP-A- 0 551 198
- EP-A- 0 856 299
- WO-A-03/057082
- US-B1- 6 254 638

## Description

La présente invention se rapporte au domaine des prothèses ou implants phonatoires ayant pour fonction la restauration de la parole chez un patient ayant subi, par exemple, une ablation totale ou partielle de la trachée et/ou du larynx. Plus particulièrement, la présente invention concerne un implant phonatoire destiné à être implanté au sein même de la paroi trachéo-oesophagienne, au travers d'un orifice ou fistule pratiqué(e) dans cette même paroi, de manière à permettre à l'air provenant de la trachée de remonter vers la bouche. Les cordes vocales n'existant plus, le patient peut ainsi moduler le son avec la langue en une voix compréhensible.

Il existe, à ce jour, diverses techniques visant à redonner l'usage de la parole à un patient ayant subi une ablation du larynx. On peut citer, à titre d'exemple, l'utilisation d'un dispositif électronique ou « éléctro-larynx ». Un tel dispositif, outre le fait de produire un son monotone et mécanique, oblige le patient à maintenir le dispositif en place au niveau de son cou lorsqu'il parle.

Plus récemment, ont été développés plusieurs dispositifs consistant en des prothèses directement implantées au niveau de la paroi trachéo-oesophagienne. A titre d'exemples non limitatifs, on peut citer les inventions décrites dans les demandes de brevet US 4,911,716, US 4,614,516, US 4,435,853 ou encore US 5,391,205. Ces prothèses consistent en des tubes creux présentant pour la plupart, au niveau de leurs extrémités, des épaulements ou collerettes dont la fonction est de maintenir la prothèse en place au sein de la paroi trachéo-oesophagienne. Ces prothèses sont généralement constituées d'un matériau souple, comme du silicone, de manière à pouvoir plier ou tordre l'une des extrémités formant épaulement pour permette son insertion au travers de la fistule, ladite extrémité pliée ou tordue reprenant sa forme initiale de l'autre coté de la paroi. Dans la pratique, il est nécessaire de pratiquer une fistule de taille supérieure au diamètre du corps de la prothèse afin de pouvoir insérer son extrémité repliée ou tordue ce qui, outre un traumatisme plus important que nécessaire, est à l'origine de nombreux inconvénients. En effet, une fois la prothèse insérée, la liaison entre le corps de la prothèse et la paroi trachéo-oesophagienne est très médiocre du fait de l'interstice résultant de la différence de taille entre la fistule et le corps de la prothèse. Il s'en suit que la prothèse aura tendance à bouger et les tissus de la paroi trachéo-oesophagienne auront du mal à venir cicatriser en prise avec la surface externe de la prothèse et ce, d'autant, que les patients concernés sont fragilisés par les traitements de chimio et/ou radiothérapie mis en place ou rajoutés secondairement lors du geste d'ablation du larynx et des cordes vocales. En plus, cette liaison de qualité médiocre est à l'origine de nombreuses fuites de fluides, salive, eau, air, gênantes pour le patient et obligeant souvent à l'ablation définitive de la prothèse, avec parfois nécessité d'effectuer un geste chirurgicale de fermeture. Un autre inconvénient provient de l'accumulation de fluides dans cet interstice, obligeant ainsi à retirer la prothèse afin de la nettoyer ou bien la remplacer, avec tous les risques liés à une telle opération.

On peut également citer la demande de brevet EP 0 651 980 qui décrit une prothèse dont l'objectif est de limiter les flux de fluides ou de nourriture provenant de l'oesophage vers la trachée. Pour ce faire, l'invention propose de disposer des valves ou clapets anti-retour. Il n'en reste pas moins vrai que les inconvénients mentionnés plus haut persistent.

Un autre perfectionnement est proposé dans la demande de brevet WO 96/03095 qui décrit le recours à l'utilisation de pigments opaques au sein même de la résine silicone utilisée pour la fabrication de la prothèse. L'intérêt, aux dires du déposant, serait de permettre de vérifier le positionnement ou la déformation de la prothèse plus facilement, par rayons X par exemple, et donc ainsi d'éviter tout changement non nécessaire. Si cette demande de brevet apporte effectivement un avantage indéniable, il n'en reste pas moins vrai qu'elle ne règle en pratique aucunement les inconvénients mentionnés plus haut.

Conscients que la plupart des inconvénients découlent du fait de la nécessité de pratiquer une fistule au sein de la paroi trachéo-oesopahgienne de taille supérieure au diamètre de la prothèse, différents dispositifs ont été développés pour permettre l'insertion de la prothèse tout en limitant au maximum la taille de l'orifice pratiqué. Une première alternative est décrite dans la demande de brevet WO 03/057082 et consiste à réaliser la collerette de rétention en alternant des épaisseurs plus ou moins importantes, ce qui facilite le pliage de la collerette et permet ainsi de diminuer la taille en position repliée, de l'extrémité à insérer. Si ce perfectionnement représente un certain progrès, il ne règle que partiellement le problème, à savoir qu'il est quand même nécessaire d'insérer une extrémité repliée, donc de taille sensiblement plus importante que le corps de la prothèse, dans la fistule.

Une autre alternative, plus répandue, consiste en un dispositif se présentant sous la forme d'une capsule ou d'un obus dans lequel on vient loger l'extrémité de la prothèse replié. Si l'utilisation d'un tel dispositif facilite effectivement l'introduction de la prothèse dans la fistule, il ne permet pas de réduire considérablement le diamètre de la fistule. La demande de brevet EP 0 551 198 décrit un tel dispositif d'insertion présentant, en outre, la propriété d'être soluble dans les fluides présents dans l'oesophage. Ici encore, cette propriété permet de faciliter le retrait du dispositif mais ne permet pas de réduire la taille de la fistule pratiquée dans la paroi trachéo-oesophagienne et, de ce fait, ne permet pas de pallier aux inconvénients décrits plus haut.

Enfin, un autre inconvénient bien connu des praticiens est la colonisation fongique, principalement par *Candida Albicans*, du silicone constituant la prothèse. En effet, la présence d'un tel interstice en milieu aérobie, humide et dans le noir, constitue un terrain propice au développement de bactéries ou champignons. Une solution utilisée à ce jour consiste en l'application d'un antifongique local de type Nystatin® ou encore Mycelex®. Pour être efficaces, ces antifongiques nécessitent d'être appliqués au moins deux fois par jour, ce qui implique des manipulations de la part du patient, et donc non seulement un certain inconfort mais également des risques de déplacement ou de mauvais repositionnement de la prothèse. Cependant, même combinée à des traitements antifongiques la durée d'une prothèse en silicone ne dépasse pas six mois, rendant impérieux son changement périodique. La réitération du geste chirurgical décuple les problèmes de cicatrisation et d'augmentation de la taille de la fistule décrits ci-dessus.

Par conséquent, il existe à ce jour un réel besoin de disposer d'une prothèse permettant de rétablir un certain usage de la parole et de pallier à l'ensemble des inconvénients décrits plus haut.

La présente invention se propose de pallier à l'ensemble des inconvénients décrits et propose un implant phonatoire totalement innovant et reposant sur un tout nouveau concept inventif jusqu'à aujourd'hui jamais décrit ni même suggéré dans l'art antérieur.

En effet, l'objectif premier de la demanderesse est de fournir un implant phonatoire ayant une tenue irréprochable au sein de la paroi trachéo-oesophagienne et dont le corps est en contact direct avec ladite paroi, permettant ainsi d'empêcher tout risque de déplacement de l'implant, de fuite d'air ou bien encore d'accumulation de fluide ou de nourriture au niveau de la paroi trachéo-oesophagienne.

Pour ce faire, la demanderesse a développé l'implant selon la revendication 1. Cet implant va à l'encontre de l'ensemble des préjugés et des recherches actuelles qui tendent, comme décrit plus haut dans l'art antérieur, à réduire au maximum la rigidité de la prothèse afin de pouvoir la replier le plus possible sur elle-même. La présente invention explore une toute autre alternative puisqu'elle envisage, pour la première fois, l'utilisation d'un implant rigide constitué de plusieurs parties.

Selon une première forme de réalisation la présente invention consiste en un implant phonatoire (1) constitué d'une portion centrale (3) formant corps cylindrique creux présentant au niveau de chacune de ses extrémités une portion latérale (5, 7) de circonférence supérieure formant épaulement ou collerette, ledit implant (1) venant se positionner dans un orifice ou fistule traversant de part en part la paroi trachéo-oesophagienne (9), chaque portion latérale (5, 7) venant en butée au niveau respectivement des faces internes des parois de la trachée (13) et de l'oesophage (11) afin de maintenir l'implant (1) en place, ledit implant phonatoire (1) étant composé d'au moins deux parties (A, B), l'une des au moins deux parties étant dite partie mobile (B), et chaque portion latérale (5, 7) étant disposée sur une partie différente de l'implant (1) de manière à permettre l'insertion de l'implant par un orifice de taille réduite correspondant au diamètre de la portion centrale, ladite partie mobile étant par la suite rendue solidaire de la portion centrale au moyen d'un élément de liaison in situ. Les parties au contact des muqueuses (25) sont en titane micro-poreux.

Par corps cylindrique creux, il faut comprendre toute géométrie de forme générale sensiblement cylindrique, c'est-à-dire sans forme anguleuse. Toute forme « en partie cylindrique » ou présentant une surface sensiblement arrondie devra être considérée comme une forme équivalente et donc comprise dans l'étendue de la protection conférée par la présente demande de brevet.

Dans la pratique, le diamètre de la partie à insérer correspond au diamètre de la partie centrale formant corps cylindrique. L'épaulement formant butée, correspondant à la deuxième partie de l'implant, n'est plus à insérer par la fistule, mais vient se positionner une fois l'implant inséré. Par conséquent, en utilisant l'implant objet de l'invention, il n'est plus nécessaire de pratiquer une fistule de taille suffisante, c'est-à-dire supérieure à celle du corps même de l'implant, pour pouvoir introduire l'une des extrémités de l'implant repliée sur elle-même, mais une fistule de taille correspondante au diamètre réel du corps de l'implant suffit.

Selon une première forme de réalisation, dans laquelle l'implant est en deux parties, la partie constituée de la portion centrale avec, au niveau de l'une de ses extrémités, une portion latérale est introduite au travers de la fistule jusqu'à ce que la première portion latérale vienne en butée au niveau de la paroi trachéo-oesophagienne. Il ne reste plus alors au chirurgien qu'à venir fixer, sur l'extrémité libre la seconde partie consistant en la seconde portion latérale.

Selon une autre forme de réalisation de l'invention, l'implant peut être réalisé en plusieurs parties comme, à titre d'exemple non limitatif, en trois parties correspondant respectivement à la portion centrale et aux deux portions latérales.

Afin de pouvoir rendre solidaires les différentes parties de l'implant selon l'invention, il peut être prévu des éléments de liaisons adéquates. Ces éléments de liaisons seront décrits plus bas.

Toutefois, selon une première forme d'exécution préférée de l'invention, l'implant phonatoire est **caractérisé en ce que** ladite partie mobile (B) consiste en un disque venant se fixer directement au niveau de l'extrémité libre (15) de la portion centrale (3).

Selon une seconde forme d'exécution encore plus préférée de l'invention, la partie mobile de l'implant phonatoire (B) comprend un corps cylindrique creux (17) de diamètre (d) inférieure au diamètre (D) du corps cylindrique creux formant la portion centrale (3) de l'implant (1) de manière à pouvoir venir coulisser au sein de ce dernier.

Un avantage de cette forme de réalisation est qu'il peut être envisagé de ne pas prévoir d'éléments de liaison. En effet, la partie mobile étant dimensionnée de manière à venir s'encastrer dans le corps cylindrique creux de l'implant, le maintien des deux parties peut n'être alors assuré que par les forces de frottement entre les deux parties. Néanmoins, il reste préférable de prévoir en sus des éléments de liaisons.

Par éléments de liaisons, il faut comprendre tout moyen permettant d'assurer le maintient de la partie mobile sur la partie fixe. De manière préférée, il doit être prévu un moyen de liaison non définitif, c'est-à-dire que le chirurgien, par exemple, doit pouvoir désolidariser les différentes parties de l'implant.

A titre d'exemples non limitatifs, on peut envisager des éléments de liaison de type vis, écrous ou tout moyen mécanique de la sorte. Il peut également être envisagé des moyens de « clipsage » ou bien des moyens électromagnétiques comme des aimants de polarité inverse.

Plus particulièrement, selon une forme de réalisation préférée, l'implant phonatoire (1) est caractérisé en ce que l'élément de liaison de la partie mobile (B) à la portion centrale (3) consiste en un filetage femelle (18) et une vis filetée imperdable portant un filetage mâle (19) respectivement au niveau de l'extrémité libre (15) de la portion centrale (3) et de la base (23) de la portion latérale mobile (B).

Comme abordé plus haut, l'implant objet de la présente invention se distingue également de l'art antérieur en ce sens qu'il est constitué d'un matériau rigide. L'utilisation en soi d'un tel matériau est innovante car elle va à l'encontre même des efforts développés à ce jour, comme le démontrent les inventions de l'art antérieur qui visent, à l'opposé, à diminuer au maximum la rigidité des implants (voir par exemple la demande de brevet WO 03/057082).

Plus particulièrement, l'implant phonatoire (1) selon l'invention est préférentiellement constitué d'un matériau rigide inerte sur le plan biologique

De tels matériaux inertes sur le plan biologiques peuvent consister en, par exemple, du verre poreux, du corail, dé la poudre d'os, de la mousse de titane utilisant de l'hydrure de titane.

Selon une forme d'exécution préférée, l'implant est constitué au moins en partie par un matériau métallique biocompatible, préférentiellement le titane et plus préférentiellement le titane microporeux.

Le titane microporeux est composé d'une juxtaposition tridimensionnelle de billes de titane non allié (voir norme NF ISO 5832-2 de mars 1997) obtenu selon le procédé décrit dans la demande de brevet EP 0856 299 A1. Selon ce procédé les prothèses métalliques présentent une porosité ouverte qui se caractérisent par des espaces intersphéroïdaux présentant une dimension sensiblement égale au tiers du diamètre des poudres, comprises donc entre 50 et 150MM pour des poudres de 150 à 500 µm.

Le procédé de préparation des prothèses métalliques selon l'un des modes de réalisation décrit dans le brevet EP 0 856 299 A1 implique la mise en oeuvre des diverses étapes successives suivantes :
- obtention, par pulvérisation à l'électrode tournante (ou tout autre procédé donnant des sphères avec peu ou pas d'aspérités), d'une poudre constituée par des microsphères métalliques de titane ou d'alliages à base de titane ou d'alliages biocompatibles,
- calibrage éventuel des microsphères par tamisage,
- mise en forme de la prothèse par traitement thermique visant à solidariser entre elles lesdites microsphères au niveau de leurs zones de contact mutuel, dans un moule de forme appropriée.

Comme évoqué dans ladite demande de brevet, dans le cadre d'un tel procédé, la mise en forme de la prothèse peut être réalisée par frittage thermique sous vide ou encore obtenue par électro-étincelage dans un moule de forme appropriée équipé des électrodes judicieusement disposées au travers des parois desdits moules. Les microsphères métalliques peuvent être soumises avant et/ou pendant l'opération de mise en forme à une opération de vibrage, ce qui permet d'obtenir un parfait remplissage du moule, une optimisation des propriétés mécaniques et une porosité plus homogène au travers de l'épaisseur de la prothèse obtenue.

Un autre avantage de la présente invention, résultant en partie de l'utilisation d'un matériau rigide métallique réside dans la possibilité d'utiliser plusieurs matériaux. En particulier, pour des raisons économiques par exemple, il peut être envisagé de n'utiliser le titane microporeux qu'au niveau des surfaces de l'implant directement en contact avec les tissus de la paroi trachéo-oesophagienne. En effet, l'intérêt du titane microporeux étant de permettre la colonisation tissulaire, seules les surfaces en contact avec les tissus de la paroi trachéo-oesophgagienne sont concernées.

Donc les parties de l'implant au contact des muqueuses (25) sont en titane microporeux.

Bien évidemment, cette alternative n'est aucunement limitative et il est également envisagé de pouvoir réaliser l'ensemble de l'implant en titane microporeux à l'exception de la partie recevant l'élément de liaison lorsque ce dernier nécessite un filetage. Pour les variantes utilisant une autre mode de liaison tels que la liaison par aimantation l'implant pourra être réalisé en totalité en titane microporeux.

Selon une variante de réalisation les parties de l'implant au contact des muqueuses présentent une surface bosselée ou rainurée destinée à faciliter la colonisation par les tissus de ce fait la taille des parties latérales (5,7) peut être considérablement réduite, voire supprimée.

L'implant phonatoire (1), selon une autre forme de l'invention présente au moins un clapet anti-retour (27)idéalement en feuillard de titane massif situé à l'une quelconque des extrémités de l'implant phonatoire.

Le clapet anti-retour à pour fonctions d'une part, lorsqu'il est en position fermée d'obturer l'implant pour empêcher le passage de fluides ou d'aliments de l'oesophage vers la trachée et d'autre part, de permettre lorsqu'il est en position ouverte le passage de l'air expiré par les poumons de la trachée à l'oesophage vers le siège des cordes vocales.

Pour le bonne compréhension de l'ensemble il est précisé que la partie de la trachée située au-dessus de l'implant phonatoire n'étant plus fonctionnelle, l'air inspiré par le patient circule à travers un méat pratiqué dans la trachée sensiblement en vis-à-vis de l'implant; le patient obturant ce méat à l'aide par exemple de ses doigts et expirant l'air lorsqu'il souhaite utiliser ses fonctions vocales restaurées.

Dans la pratique, il peut être envisagé de disposer plusieurs clapets anti-retour.

Selon un autre aspect de l'invention, il est envisagé un dispositif facilitant l'insertion de l'implant. Ce dispositif consiste en un « capuchon », préférentiellement en forme d'obus, venant se positionner sur l'extrémité de l'implant à insérer. Contrairement à l'art antérieur, l'utilisation d'un tel dispositif n'a pas pour objectif de maintenir une extrémité repliée en son sein, mais plutôt une fonction de confort destinée à faciliter le travail du chirurgien.

Par conséquent, il est également revendiqué le dispositif d'insertion d'un implant phonatoire (1), caractérisé en ce qu'il consiste en une capsule en forme d'obus venant recouvrir l'extrémité libre (15) de la portion centrale (3) à insérer dans la fistule et, une fois l'implant phonatoire (1) en place, étant destinée à être dégradée ou retirée de manière à permettre à la portion latérale mobile (B) de venir se fixer.

Ledit implant phonatoire (1) selon la présente invention peut être utilisé pour restaurer la fonction vocale chez un patient après une laryngectomie ou une trachéotomie.

De manière évidente, une telle utilisation n'est aucunement limitée au traitement des laryngectomies ou des trachéotomies, mais peut être étendue à tout traitement nécessitant la restauration d'un écoulement de fluides ou d'air au travers d'une paroi, préférentiellement au niveau de la paroi trachéo-oesophagienne.

L'invention sera mieux comprise, et ses avantages ressortiront plus clairement, au cours de la description suivante des deux exemples de réalisation, en référence aux dessins annexés dans lesquels :
la figure 1 est, en coupe longitudinale, une prothèse de l'art antérieur,
la figure 2 représente, en coupe longitudinale, une forme de réalisation de l'invention,
la figure 3 représente, en coupe longitudinale, une autre forme de réalisation préférée de l'invention,
la figure 4 représente , en coupe longitudinale, un implant selon l'invention positionné au sein de la paroi trachéo-oesophagienne.

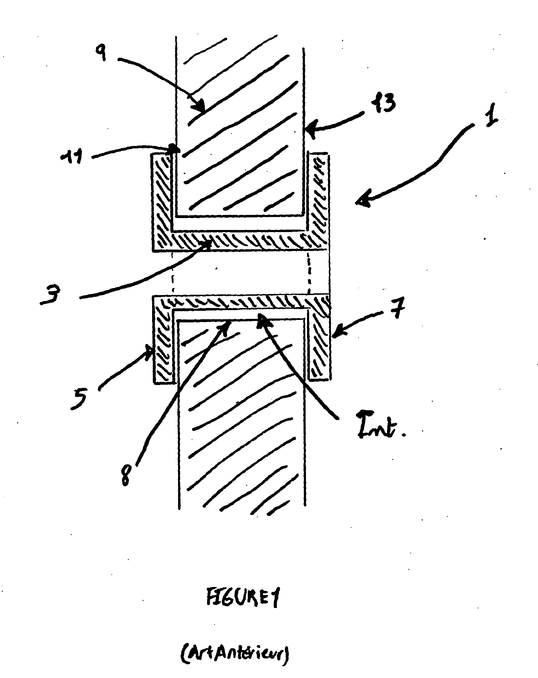

Si l'on se reporte maintenant à la figure 1, celle-ci représente une prothèse 1 de l'art antérieur constituée d'une portion centrale 3 formant corps cylindrique et de deux portions latérales 5, 7 formant collerette. Cette prothèse est réalisée en un seul tenant et en un matériau souple, type résine de silicone. Afin de pouvoir être introduite au travers de la paroi trachéo-oesophagienne 9, l'une de ses portions latérales 5, 7 a due être repliée sur elle-même afin d'en diminuer la taille et de permettre son insertion dans la fistule. Toutefois, comme explicité plus haut, ladite fistule doit avoir une taille suffisante pour permettre à cette extrémité 5, 7 repliée de pouvoir passer. Pour ce faire, la fistule doit avoir une taille supérieure à celle du diamètre de la portion centrale 3. Il en résulte un interstice Int. entre la portion centrale 3 et les bords 8 de la fistule pratiquée dans la paroi 9. Cet interstice Int. est à l'origine de fuites ainsi que d'accumulations de nourriture et/ou fluides en son sein. En outre, les tissus de la paroi trachéo-oesophagienne ne peuvent venir en contact de la prothèse et cette dernière sera susceptible de bouger au sein de la fistule.

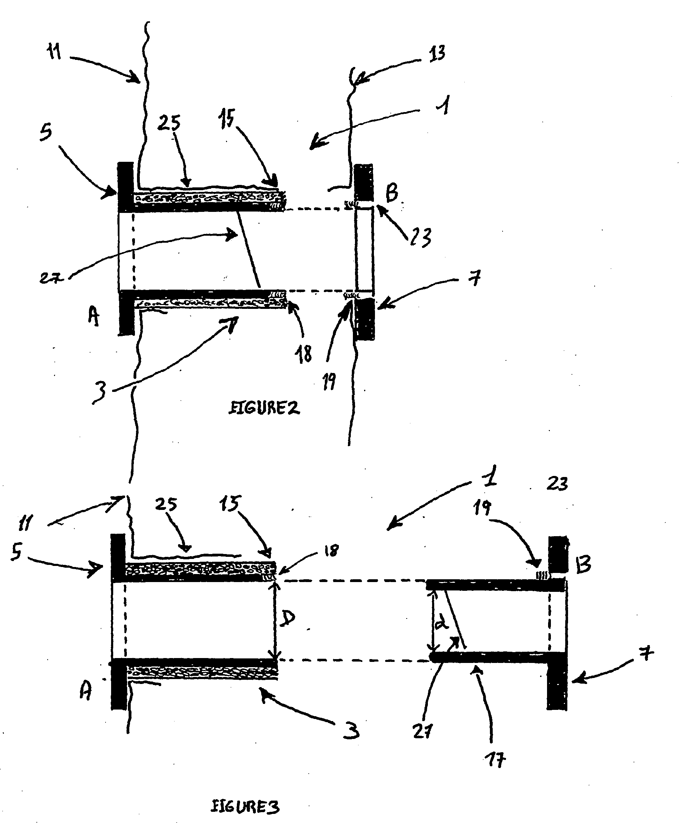

Si l'on se reporte à la figure 2, celle-ci représente, en coupe longitudinale, une première forme d'exécution non limitative de l'invention.

Plus particulièrement, il est représenté un implant phonatoire 1 selon l'invention, constitué d'une première partie A comprenant une portion centrale 3 et une portion latérale 5 et d'une seconde partie B en forme de disque et formant portion latérale 7.

La partie B, dite partie mobile, peut être rendue solidaire de la partie A par le biais du ou des élément(s) de liaison. Comme décrit plus haut, ces éléments de liaison peuvent consister en tout moyen permettant de fixer et d'immobiliser la partie B sur la partie A de manière non définitive, c'est-à-dire qu'un chirurgien, par exemple, pourra assez facilement les désolidariser. Comme vu plus haut, on peut citer un système de vis, d'aimants, de clips, ou tout système équivalent.

La figure 3 représente une seconde forme d'exécution préférée selon l'invention. L'implant 1 est constitué d'une première partie A comprenant une portion centrale 3 et une portion latérale 5 et d'une seconde partie B comprenant une portion centrale 17 formant corps cylindrique creux et d'une portion latérale 7. La portion centrale 17 de la partie B est dimensionnée de manière à pouvoir coulisser au sein de la portion centrale 3 de la partie A. Pour ce faire, la portion centrale 17 formant corps cylindrique creux a un diamètre d inférieur au diamètre D de la portion centrale 3. Dans la pratique, la partie B vient s'emboîter dans la partie A.

Dans la forme d'exécution représentée ici, il est également prévu des éléments de liaison (18, 19). Toutefois, compte tenu que les deux parties, respectivement A et B, s'emboîtent l'une dans l'autre, il pourrait être envisagé de ne pas prévoir d'élément de liaison ; le maintient résultant de par l'emboîtement même de la partie B dans la partie A.

Que ce soit dans la figure 2 ou la figure 3, il est également représenté un clapet anti-retour 27. Ce clapet à pour fonction de laisser passer l'air de la trachée vers l'oesophage et d'obstruer le passage des aliments de l'oesophage vers la trachée. Il est bien évident que ce clapet 27 pourrait être disposé autrement, au niveau de l'une ou de l'autre partie A ou B. En outre, il pourrait également être envisagé plusieurs clapets.

Enfin, il ressort également de ces figures une caractéristique propre, à savoir la disposition des différents matériaux utilisés. En effet, comme décrit plus haut, l'implant objet de l'invention est constitué d'un matériau rigide, préférentiellement métallique et encore plus préférentiellement en titane. Selon une caractéristique innovante, les parties de l'implant au contact des tissus constitutifs de la paroi trachéo-oesophagienne sont constituées d'un matériau non seulement biocompatible mais surtout colonisable comme le titane microporeux. Cette caractéristique est clairement illustrée sur les figures 2 et 3 qui représentent, en noir, les parties de l'implant qui ne sont pas en contact avec les tissus entourant la fistule et, en cercles, les parties 25 qui sont en contact avec les tissus de la paroi et donc qui sont en titane microporeux. Bien évidemment, tout matériau équivalent au titane microporeux, c'est-à-dire présentant des caractéristique de biocompatibitité et de colonisation similaires pourrait être envisagé et donc devrait être considéré comme un moyen équivalent.

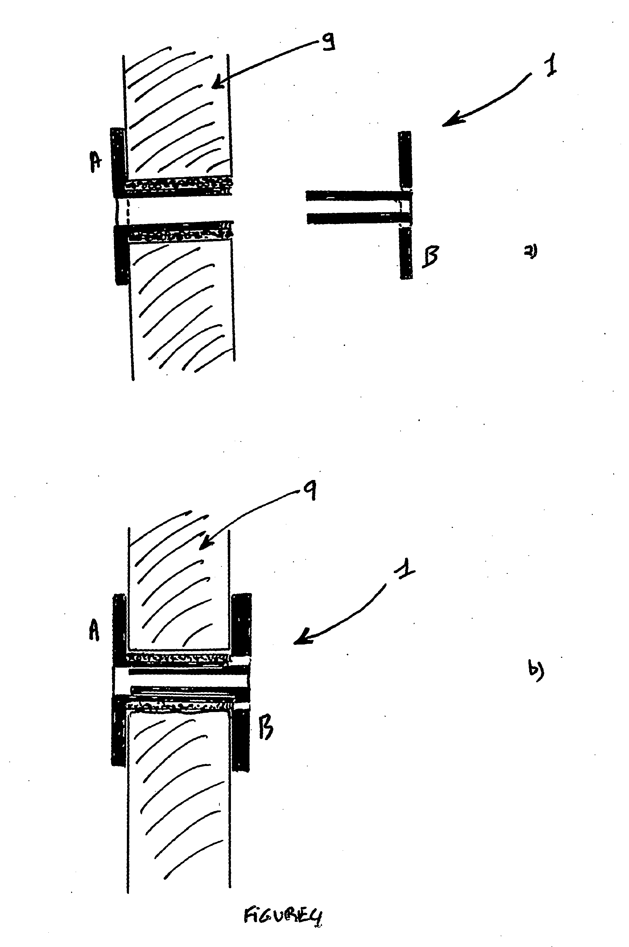

La figure 4 représente l'utilisation de l'implant selon l'invention. La figure 4a montre la partie A comprenant la portion centrale 3 insérée dans la fistule jusqu'à ce que la partie latérale 5, formant épaulement, vienne en butée de la face 11 côté oesophage de la paroi trachéo-oesophagienne 9. Comme on peut le voir, la surface externe 25 de la portion centrale 3 en contact avec les tissus de la paroi 9 est constituée de titane microporeux. Les dimensions de la portion centrale 3 formant le corps de l'implant sont déterminées de manière à ce qu'il n'y ait pas d'espace entre la surface externe de l'implant et la paroi trachéo-oesophagienne. La partie B est alors introduite par le chirurgien et vient se positionner en s'emboîtant dans la partie A. La figure 4b représente l'implant une fois installé, c'est-à-dire une fois la partie B solidarisée au sein de la partie A.

Bien évidemment, les différentes parties de l'implant selon l'invention sont dimensionnées de manière à permettre un écoulement d'air suffisant. A titre d'exemple non limitatif, le diamètre intérieur d de la partie B est préférentiellement compris entre 3 et 7 mm, préférentiellement entre 5 et 6 mm. Le diamètre D correspondant au niveau de la partie A doit être dimensionné de manière à permettre le passage de la partie A et, de préférence, doit présenter un jeu compris entre de 0,05 et 0,3 mm.

La présente description consiste en une forme générale de l'invention et toute modification ou amélioration évidente, basée sur le principe même de l'invention, doit être considéré comme équivalent.

## Revendications

1. Implant phonatoire (1) constitué d'une portion centrale (3) formant corps cylindrique creux présentant au niveau de chacune de ses extrémités une portion latérale (5, 7) de circonférence supérieure formant épaulement ou collerette, ledit implant (1) venant se positionner dans un orifice ou fistule traversant de part en part la paroi trachéo-oesophagienne (9), chaque portion latérale (5, 7) venant en butée au niveau respectivement des faces internes des parois de la trachée (13) et de l'oesophage (11) afin de maintenir l'implant (1) en place, et étant composé d'au moins deux parties (A, B), l'une des au moins deux parties étant dite partie mobile (B), et en ce que chaque portion latérale (5, 7) est disposée sur une partie différente de l'implant (1) de manière à permettre l'insertion de l'implant par un orifice de taille réduite correspondant au diamètre de la portion centrale, ladite partie mobile étant par la suite rendue solidaire de la portion centrale au moyen d'un élément de liaison in situ, ledit implant phonatoire (1) étant **caractérisé en ce que** les parties de l'implant au contact des muqueuses (25) sont en titane micro-poreux.

2. Implant phonatoire (1) selon la revendication 1, **caractérisé en ce que** ladite partie mobile (B) consiste en un disque venant se fixer directement au niveau de l'extrémité libre (15) de la portion centrale (3).

3. Implant phonatoire (1) selon la revendication 1 ou 2, **caractérisé en ce que** ladite partie mobile (B) comprend un corps cylindrique creux (17) de diamètre (d) inférieure au diamètre (D) du corps cylindrique creux formant la portion centrale (3) de l'implant (1) de manière à pouvoir venir coulisser au sein de ce dernier.

4. Implant phonatoire (1) selon l'une quelconque des revendications précédentes, **caractérisé en ce que** ledit élément de liaison de la partie mobile (B) à la portion centrale (3) consiste en un filetage femelle (18) et une vis filetée imperdable portant un filetage mâle (19) respectivement au niveau de l'extrémité libre (15) de la portion centrale (3) et de la base (23) de la portion latérale mobile (B).

5. Implant phonatoire (1) selon l'une quelconque des revendications précédentes, **caractérisé en ce qu'**il est constitué d'un matériau rigide inerte sur le plan biologique.

6. Implant phonatoire (1) selon la revendication 5, **caractérisé en ce qu'**il est constitué au moins en partie par un matériau métallique biocompatible, préférentiellement le titane.

7. Implant phonatoire (1) selon l'une quelconque des revendications précédentes, **caractérisé en ce que** les parties de l'implant au contact des muqueuses présentent une surface bosselée ou rainurée destinée à faciliter la colonisation par les tissus.

8. Implant phonatoire (1) selon l'une quelconque des revendications précédentes, **caractérisé en ce qu'**il présente au moins un clapet anti-retour (27) idéalement en feuillard de titane massif.

9. Dispositif d'insertion d'un implant phonatoire (1) en combinaison avec un implant selon l'une quelconque des revendications précédentes, **caractérisé en ce que** les parties de l'implant au contact des muqueuses (25) sont en titane micro-poreux et que ledit dispositif consiste en une capsule en forme d'obus venant recouvrir l'extrémité libre (15) de la portion centrale (3) à insérer dans la fistule et, une fois l'implant phonatoire (1) en place, étant destinée à être dégradée ou retirée de manière à permettre à la portion latérale mobile (B) de venir se fixer.

## Claims

1. Phonatory implant (1) consisting of a central portion (3) forming a hollow cylindrical body having at each of its ends a lateral portion (5, 7) of higher circumference forming a shoulder or flange, said implant (1) being urge to be positioned in an orifice or fistula running through the tracheoesophageal wall (9), each lateral portion (5, 7) abutting respectively at the inner surfaces of the walls of the trachea (13) and of the esophagus (11) so as to maintain the implant (1) in position, and consisting of at least two parts (A, B), at least one of the two parts being called mobile part (B), and in which each lateral portions (5, 7) is arranged on a different part of the implant (1) so as to enable the implant to be inserted through an orifice of reduced size corresponding to the diameter of the central portion, said mobile part being made interdependent with the central portion using an in situ linking element, said phonatory implant (1) being **characterized in that** the parts of the implant (1) in contact with the mucous membranes (25) are made of micro-porous titanium.

2. Phonatory implant (1) according to claim 1, **characterized in that** said mobile part (B) consists of a disk directly fixed at the level of the free end (15) of the central portion (3).

3. Phonatory implant (1) according to claim 1 or 2, **characterized in that** said mobile part (B) comprises a hollow cylindrical body (17) having a diameter (d) lower than the diameter (D) of the hollow cylindrical body forming the central portion (3) of the implant (1) so as to be able to slide within the latter.

4. Phonatory implant (1) according to any preceding claim, **characterized in that** said linking element being of the mobile part (B) to the central portion (3) consists of a female thread (18) and a captive threaded screw bearing a male thread (19) respectively at the level of the free end (15) of the central portion (3) and at the base (23) of the mobile lateral portion (B).

5. Phonatory implant (1) according to any preceding claim, **characterized in that** it is made of a rigid material that is inert at a biological level.

6. Phonatory implant (1) according to claim 5, **characterized in that** it is at least in part made of a metallic biocompatible material, and preferentially titanium.

7. Phonatory implant (1) according to any preceding claim, **characterized in that** the parts of the implant in contact with the mucous membranes have a embossed or grooved surface intended to facilitate the colonisation by tissues.

8. Phonatory implant (1) according to any preceding claim, **characterized in that** it presents at least one backflow-preventing valve (27) ideally made of a massive titanium strip.

9. Device for the insertion of a phonatory implant (1) in combination with an implant according to any preceding claim, **characterized in that** the parts of the implant in contact with the mucous membranes (25) are made of micro-porous titanium and that said device consists of a shell-shaped capsule covering the free end (15) of the central portion (3) to be inserted within the fistula and, once the phonatory implant (1) is in place, intended to be degraded or to be removed in order to enable the lateral mobile portion (B) to fix itself.

## Patentansprüche

1. Stimmprothesenimplantat (1) betstehend aus einem Mittelstück (3), welches einen zylindrischen Hohlkörper bildet, der an jedem seiner beiden Enden ein Seitenstück (5, 7) größeren Umfangs aufweist und somit einen Vorsprung oder Schild ausbildet, wobei besagtes Implantat (1) in eine Öffnung bzw. Fistel eingesetzt wird, welche von einer Seite der ösophagotrachealen Wand (9) zur anderen verläuft, wobei jedes Seitenstück (5, 7) jeweils auf den Innenwänden der Trachea (13) und des Ösophagus (11) aufliegt, um das Implantat (1) in seiner Position zu fixieren um ein Dislozieren des Implantates (1) zu verhindern, und welches aus mindestens zwei Teilen besteht (A, B), wobei einer der mindestens zwei Teile als beweglicher Teil (B) bezeichnet wird und jedes Seitenstück (5, 7) auf einem anderen Teil des Implantats (1) angeordnet ist, so dass das Implantat durch eine Öffnung geringerer Größe eingeführt werden kann, welche dem Durchmesser des Mittelstücks entspricht, wobei besagter beweglicher Teil anschließend anhand eines Verbindungsstücks in situ mit dem Mittelstück verfestigt wird, wobei sich besagtes Implantat (1) **dadurch kennzeichnet, dass** die mit Schleimhäuten in Kontakt stehenden Prothesenteile (25) aus mikroporösen Titan bestehen.

2. Stimmprothesenimplantat (1) nach Anspruch 1, **dadurch gekennzeichnet, dass** besagter beweglicher Teil (B) aus einer Scheibe besteht, welche direkt auf dem freie Ende (15) des Mittelstücks (3) befestigt wird.

3. Stimmprothesenimplantat (1) nach Anspruch 1 oder 2, **dadurch gekennzeichnet, dass** besagter bewegliche Teil (B) einen zylindrischen Hohlkörper umfasst (17) dessen Durchmesser (d) kleiner ist als der Durchmesser (D) des zylindrischen Hohlkörper, welcher das Mittelstück (3) des Implantats (1) bildet, so dass er in letzteren hineingeschoben werden kann.

4. Stimmprothesenimplantat (1) nach einem beliebigen vorstehenden Anspruch, **dadurch gekennzeichnet, dass** das besagte Verbindungsstück zwischen dem beweglichen Teil (B) und dem Mittelstück (3) jeweils am freien Ende (15) des Mittelstücks (3) und am Ansatz (23) des bewegliche Seitenteils (B) ein Innengewinde (18) und eine unverlierbare Gewindeschraube mit Außengewinde (19) umfasst.

5. Stimmprothesenimplantat (1) nach einem beliebigen vorstehenden Anspruch, **dadurch gekennzeichnet, dass** diese aus einem festen, aus biologischer Sicht inerten Material besteht.

6. Stimmprothesenimplantate (1) nach Anspruch 5, **dadurch gekennzeichnet, dass** diese mindestens zu einem Teil aus einem biokompatiblen, metallischen Material besteht, vorzugsweise aus Titan.

7. Stimmprothesenimplantate (1) nach einem beliebigen vorstehenden Anspruch, **dadurch gekennzeichnet, dass** die mit der Schleimhaut in Kontakt stehenden Teile des Prothese eine gedellte oder geriffelte Oberfläche aufweisen, um das Anwachsen des Gewebes zu erleichtern.

8. Stimmprothesenimplantate (1) nach einem beliebigen vorstehenden Anspruch, **dadurch gekennzeichnet, dass** diese mindestens ein Rückschlagventil (27) aufweist, das Idealerweise aus massivem Titan-Band besteht.

9. Implantationsinstrument für Stimmprothesenimplantate (1) in Kombination mit einem Implantat nach einem beliebigen vorstehenden Anspruch, **dadurch gekennzeichnet, dass** die mit der Schleimhaut in Kontakt stehenden Prothesenteile (25) aus mikroporöse Titan bestehen und dass besagtes Instrument aus einer projektilförmigen Kapsel besteht, welche das freie Ende (15) des in die Fistel einzuführenden Mittelstücks (3) umhüllt, und dessen Zerstörung oder Entfernung nach Einsetzen des implantierbaren Stimmprothese (1) vorgegehen ist, um die Befestigung des mobilen Seitenteils (B) zu ermöglichen.
